(19) Europäisches Patentamt
European Patent Office
Office européen des brevets

(11) Publication number: **0 294 695**
**A2**

(12) # EUROPEAN PATENT APPLICATION

(21) Application number: **88108743.1**

(51) Int. Cl.4: **C07D 311/92 , A61K 31/35**

(22) Date of filing: **01.06.88**

Patentansprüche für folgende Vertragsstaaten: ES + GR.

(30) Priority: **06.06.87 DE 3719054**
**12.09.87 DE 3730748**

(43) Date of publication of application:
**14.12.88 Bulletin 88/50**

(84) Designated Contracting States:
**AT BE CH DE ES FR GB GR IT LI LU NL SE**

(71) Applicant: **HOECHST AKTIENGESELLSCHAFT**
**Postfach 80 03 20**
**D-6230 Frankfurt am Main 80(DE)**

(72) Inventor: **Khandelwal, Yatendra, Dr.**
**M-8, Hoechst Executive Quarters Darga Road**
**Mulund (West) Bombay 400 082(IN)**
Inventor: **Kannan, Rajeshwari**
**1st floor, Abhinandan Apartments**
**Sarvodaya Nagar Mulund Bombay 400 080(IN)**
Inventor: **Lal, Bansi, Dr.**
**30 Adavani Apartments Mulund(West)**
**Bombay 400 080(IN)**
Inventor: **Aroskar, Vijay A. 5, Asha Kiran Co. Housing Soc**
**Plot No. 17-C, Linking Road Extn.**
**Santa Cruz(West) Bombay 400 054(IN)**
Inventor: **Dohadwalla, Alihussein Nomanbhai, Dr.**
**Noman Mansion 139 c. Cumballa Hill**
**Bombay 400 036(IN)**
Inventor: **Rupp, Richard Helmut, Dr.**
**Roederweg 16a**
**D-6240 Königstein/Taunus(DE)**

(54) **New polyoxygenated labdane derivatives, a process for their preparation, and their use as medicaments.**

(57) The present invention relates to polyoxygenated labdane derivatives of the formula

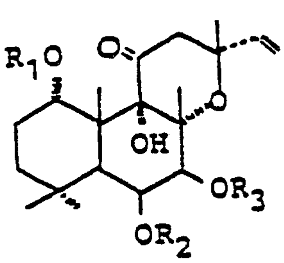

(I)

a process for their preparation and the use of these substances as medicaments, acting especially against elevated intraocularpressure, elevated blood pressure, congestive heart failure, bronchialasthma and inflammations.

**EP 0 294 695 A2**

## New polyoxygenated labdane derivatives, a process for their preparation, and their use as medicaments

The present invention relates to new polyoxygenated labdane derivatives and to a process for their preparation. The compounds according to the invention have valuable pharmacological properties and can thus be used as medicaments.

The new compounds according to the invention correspond to the general formula I

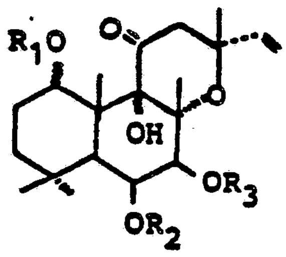

I

in which $R_1$, $R_2$ and $R_3$ are identical or different and denote:
hydrogen, $C_1$-$C_6$-alkyl, $C_2$-$C_6$-alkanoyl or a radical of the formula
$-\overset{Z}{\overset{\|}{C}}-A$, in which Z represents oxygen or sulfur, and A either represents the radical

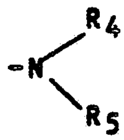

in which $R_4$ represents hydrogen or $C_1$-$C_6$-alkyl, and $R_5$ represents $C_1$-$C_6$-alkyl, $C_3$-$C_7$-cycloalkyl, aryl, aryl-$C_1$-$C_6$-alkyl, $C_2$-$C_6$-carbalkoxy or sulfonylaryl, or $R_4$ and $R_5$, together with the nitrogen atom to which they are bonded, form a heterocycle which may contain as further heteroatom oxygen, nitrogen or sulfur, or A represents the radical $-OR_6$ in which $R_6$ represents $C_1$-$C_6$-alkyl or halogeno-$C_1$-$C_6$-alkyl, $R_1$ represents a tris($C_1$-$C_6$-alkyl)silyl group, and $R_2$ and $R_3$ have the said meanings,
with the proviso that
1. $R_1$, $R_2$ and $R_3$ do not simultaneously represent hydrogen, and
2. at least one of the three substituents $R_1$, $R_2$ and $R_3$ represents the radical $-\overset{Z}{\overset{\|}{C}}-A$ when the other substituent(s) represents hydrogen, $C_1$-$C_6$-alkyl or $C_1$-$C_6$-alkanoyl,
and its pharmacologically acceptable acid addition salts and optical and geometric isomers.

The compounds according to the invention can be used as medicaments acting against elevated intraocular pressure, elevated blood pressure, congestive heart failure, bronchial asthma and inflammations.

Subgroups of compounds according to the invention, of the formula I, are those in which

a) $R_1$ represents the radical $-\overset{Z}{\overset{\|}{C}}-A$, Z and A having the abovementioned meanings, $R_2$ denotes hydrogen, and $R_3$ denotes hydrogen or $C_1$-$C_6$-alkanoyl,

b) $R_1$ denotes hydrogen or represents the radical $-\overset{Z}{\overset{\|}{C}}-A$, in which Z and A have the said meanings, $R_2$ represents hydrogen or $C_1$-$C_6$-alkanoyl, and $R_3$ represents the radical $-\overset{Z}{\overset{\|}{C}}-A$,

Z and A having the said meanings,

c) $R_1$ represents hydrogen or the radical $-\overset{\overset{Z}{\|}}{C}-A$, Z and A having the said meanings,

$R_2$ represents the radical $-\overset{\overset{Z}{\|}}{C}-A$ ,
Z and A having the said meanings, and
$R_3$ denotes hydrogen or $C_1$-$C_6$-alkanoyl.

In the definitions used above, $C_1$-$C_6$-alkyl denotes a straight-chain or branched hydrocarbon group having 1 to 6 carbon atoms, such as methyl, ethyl, 1-propyl, 1-butyl, 2-pentyl or 3-hexyl; aryl denotes a phenyl group which is optionally substituted by $C_1$-$C_6$-alkyl, $C_1$-$C_6$-alkoxy, halogen or trifluoromethyl; halogen denotes a fluorine, chlorine, bromine or iodine atom; $C_2$-$C_6$-carbalkoxy denotes groups such as carbethoxy or carbomethoxy; sulfonylaryl denotes a phenylsulfonyl group, phenyl optionally being substituted as indicated above. Where $R_4$ and $R_5$, together with the nitrogen atom to which they are bonded, represent a heterocycle, examples of possible heterocycles are piperidine, piperazine, morpholine or thiomorpholine, each of which can be substituted by optionally substituted $C_1$-$C_6$-alkyl, preferably $C_1$-$C_3$-alkyl, or by an aryl group.

Where $R_1$, $R_2$, $R_3$, $R_4$ or $R_5$ denotes alkyl, alkanoyl or carbalkoxy groups, those having 1-4 carbon atoms are preferred.

Preferred cycloalkyl groups for $R_5$ are cyclopentyl and cyclohexyl.

In the formulae, the various substituents are shown with one of two types of bonding to the labdane nucleus: a full line ( _______ ) which indicates that a substituent is in the $\beta$-orientation (i.e. above the plane of the molecule), and a broken line (---) which indicates that a substituent is in the $\alpha$-orientation (i.e. below the plane of the molecule). All the formulae have been drawn in such a way that they show the compounds in their absolute stereochemical configuration. Where the starting materials having a labdane nucleus occur naturally, or are derived from naturally occurring substances, both these and the final produces have a labdane nucleus in the only absolute configuration depicted here. However, the process according to the invention also relates to the synthesis of labdanes of the racemic series.

Besides the optical centers of the labdane nucleus, the substituents thereon may likewise have chiral centers which contribute to the optical properties of the compound according to the invention and allow the separation thereof by conventional methods, for example by use of optically active acids. Where a wavy line (~) connects a group to a chiral center, this means that the stereo-chemistry of the center is unknown, i.e. the group may be present in either of the possible orientations. This invention embraces all the optical isomers and racemic forms of the compounds according to the invention where such compounds contain additional chiral centers besides those of the labdane nucleus.

Some of the new polyoxylabdane derivatives according to the invention are listed in Table 1 which follows.

Table 1

| Compound Nr. | $R_1$ | $R_2$ | $R_3$ | Melting point (°C) |
|---|---|---|---|---|
| 1 | H | H | $CONHCH_3$ | 129-30 |
| 2 | H | H | $CSNHCH_3$ | 223-24 |
| 4 | H | H | $CONHCH(CH_3)_2$ | 120-24 |
| 5 | H | H | $CONHC(CH_3)_3$ | 123-25 |
| 6 | H | H | CONH 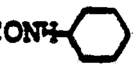 | 174-75 |
| 7 | H | H | $CSNHCH_2C_6H_5$ | 203-05 |
| 8 | H | H | CON 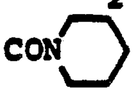 | 198-99 |
| 9 | H | H | $CONHC_6H_5$ | 168 |
| 10 | H | $CONHC_6H_5$ | H | 169-72 |
| 11 | CONH- 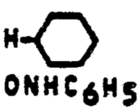 | H | $COCH_3$ | 194-95 |
| 12 | $CONHC_6H_5$ | H | $COCH_3$ | 223-25 |
| 15 | H | H | p-Chlor-$C_6H_4NHCO$ | 194-95 |
| 16 | H | H | p-$CF_3$-$C_6H_4NHCO$ | 139-42 |
| 17 | H | H | $CONHSO_2C_6H_5$ | 154-56 |
| 18 | H | H | $CONHSO_2C_6H_4$-p-$CH_3$ | 184-85 |
| 19 | H | H | $CON(C_2H_5)_2$ | 216-19 |
| 20 | $CON(C_2H_5)_2$ | H | $COCH_3$ | 158-59 |
| 21 | $CONHC_6H_5$ | H | $CONHC_6H_5$ | 234-37 |
| 22 | H | H | $COOC_2H_5$ | 160-61 |
| 23 | H | H | $COOCH_2CCl_3$ | 169 |
| 24 | H | H | $CONHCH_2Ph$ | 201-03 |
| 25 | H | H | $CON(CH_3)-CONHCH_3$ | 218-19 |

The process for the preparation of compounds of the formula I, in which at least one of $R_1$, $R_2$ and $R_3$ denotes

the group $-\overset{Z}{\overset{\|}{C}}-A$, in which A represents

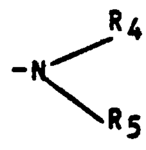

and Z represents O or S, comprises initially reacting a compound of the formula II

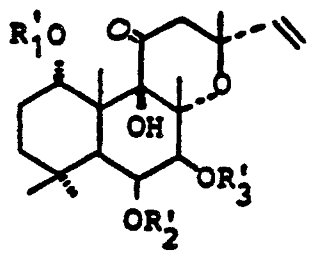

II

in which $R_1'$, $R_2'$ and $R_3'$ all denote hydrogen (i.e. 7-deacetylforskolin) or $R_1'$ and $R_2'$ denote hydrogen, and $R_3'$ denotes acetyl (i.e. forskolin) or $R_1'$ denotes alkylsilyl such as, for example, a t-butyldimethylsilyl group, and $R_2'$ and $R_3'$ denote hydrogen, with a compound of the formula III

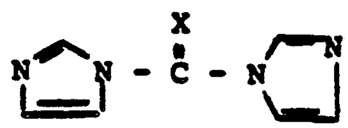

III

in which X represents oxygen or sulfur, and then adding to the reaction mixture, at room temperature and maintaining an inert atmosphere using, for example, nitrogen gas, an amine of the formula

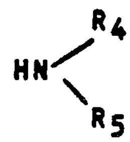

in which $R_4$ and $R_5$ have the above meanings, in organic solvents such as methyl acetate, stirring for a total period of 26 to 36 hours, where appropriate eliminating the protective group such as alkylsilyl in the 1-position, and obtaining the compounds of the formula I in a known manner, for example as described in the example, from the reaction mixture, and purifying them. It is also possible to prepare compounds of the formula I by reacting the compounds of the formula II described above with an isocyanate of the formula RNCX, in which R represents cycloalkyl or aryl, and X represents oxygen or sulfur, for 15 to 36 hours under reflux in organic solvents such as pyridine, in the presence of a catalyst such as 4-dimethylaminopyridine, where appropriate eliminating a protective group such as alkylsilyl in the 1-position, and obtaining and purifying the product in a known manner, as described in the examples.

The process for the preparation of compounds of the formula I in which at least one of $R_1$, $R_2$ and $R_3$ represents a group of the formula $-\overset{Z}{\overset{\|}{C}}-OR_6$, Z denoting oxygen, and $R_6$ denoting alkyl or substituted alkyl, such as halogenoalkyl, comprises reacting compounds of the formula II, initially at the temperature of ice and then at room temperature, with compounds of the formula $Cl-\overset{O}{\overset{\|}{C}}-OR_6$, in which $R_6$ has the same meaning as above, in organic solvents such as pyridine, under a nitrogen atmosphere, where appropriate eliminating the protective group such as alkylsilyl in the 1-position, and obtaining and purifying the product in a known manner, for example by extraction and purification by chromatography as described in the examples.

In the case of compounds of the formula II in which $R_1$ is an alkylsilyl protective group such as t-butyldimethylsilyl, the protective group is removed from the resulting compounds of the formula I by

treatment with, for example, tetrabutylammonium fluoride in an organic solvent such as tetrahydrofuran, in order to obtain the corresponding compound of the formula I with $R_1$ = H. If desired, it is possible to rearrange compounds of the formula I with $R_2$ = H and $R_3$ = $-\overset{Z}{\overset{\|}{C}}-A$, in which Z and A have the above meanings, by use of an alkali metal alcoholate in an alkanol or ether solvent, alone or in a mixture of the latter, at room temperature, and to obtain and purify the product in a known manner, for example by extraction and column chromatography and/or crystallization.

The starting compounds of the formula II, i.e. forskol in and 7-deacetylforskolin, are known from the literature (Tetrahedron Letters No. 19, pp. 1169-72, 1977; J. Chem. Soc., Perkin Trans., 1, 767, 1982), and 1α-t-butyldimethylsilyl-8,13-epoxy-6β,7β,9α-trihydroxylabd-14-en-11-one is prepared as described in German Patent Application P 36 23 305 (HOE 86/F 153).

The results of investigations into the reduction of intraocular pressure by some typical compounds of the formula I, according to the invention, are listed in Table 2. The results are expressed as the percentage decrease in the intraocular pressure when a 2% strength solution of the test compound is used.

Table 2

| Compound Nr. | $R_1$ | $R_2$ | $R_3$ | Decrease in intraocular pressure(%) | Duration in hours |
|---|---|---|---|---|---|
| 1 | $CONHC_6H_5$ | H | $CONHC_6H_5$ | 18 | 5 |
| 2 | H | H | $CONHCH_3$ | 23 | 5 |
| 3 | H | H | $CONHCH(CH_3)_2$ | 34 | 6 |
| 4 | H | H | $CONHC(CH_3)_3$ | 21 | 6 |

The examples which follow illustrate the invention, without restricting its scope:

Example 1

1α-t-Butyldimethylsilyl-8,13-epoxy-6β,9α-dihydroxy-7β-piperidinocarbonyloxylabd-14-en-11-one

Under nitrogen, 1,1′-carbonyldiimidazole (0.373 g, 2.3 mmol) is added to a stirred solution of 1α-t-butyldimethylsilyl-8,13-epoxy-6β,7β,9α-trihydroxylabd-14-en-11-one (1.0 g, 2.07 mmol) in dry ethyl acetate (25 ml). The mixture is stirred at room temperature overnight, a further quantity of 1,1′-carbonyldiimidazole (0.17 g, 1.65 mmol) is added, and the mixture is then stirred at room temperature for a further 12 hours.

Piperidine (1 ml) is added, and the mixture is then stirred for one hour, diluted with more ethyl acetate, washed with water, dried over anhydrous sodium sulfate and concentrated. The residue is purified by flash column chromatography using chloroform:diisopropyl ether:petroleum ether (1:1:2) as eluent, the product being obtained in 40% yield.

Similarly the following compounds were prepared:

8,13-Epoxy-7β-methylaminocarbonyloxy-1α,6β,9α-trihydroxylabd-14-en-11-one, m.p. 129-30° C.

7β-Benzylaminocarbonyloxy-8,13-epoxy-1α,6β,9α-trihydroxylabd-14-en-11-one, m.p. 201-03° C.

Example 2

8,13-Epoxy-7β-piperidinocarbonyloxy-1α,6β,9α-trihydroxylabd-14-en-11-one

Tetrabutylammonium fluoride trihydrate (0.25 g) is added to a solution of 1α-t-butyldimethylsilyl-7β-piperidinocarbonyloxy-6β,9α-dihydroxy-8,13-epoxylabd-14-en-11-one (0.43 g, 0.725 mmol) in THF (15 ml), the mixture is stirred at room temperature for 10 min and then concentrated in vacuo. The residue is extracted with ethyl acetate, and the organic layer is washed with water, dried over anhydrous sodium sulfate and concentrated. The residue is purified by flash column chromatography using ethyl acetate:petroleum ether (45:55) as eluent. The resulting product is crystallized from an ethyl acetate/petroleum ether mixture: yield 60%, melting point 198-199 °C.

Example 3

7β-t-Butylaminocarbonyloxy-8,13-epoxy-1α,6β,9α-trihydroxylabd-14-en-11-one

t-Butyl isocyanate (1.68 ml, 19.5 mmol) is added to a solution of 1α-t-butyldimethylsilyl-8,13-epoxy-6β,7β,9α-trihydroxylabd-14-en-11-one (0.7 g, 1.45 mmol) in pyridine (15 ml), and the mixture is heated under reflux for 15 hours, following the reaction by thin-layer chromatography (TLC). After the starting material has disappeared on the TLC, the reaction mixture is poured onto ice, and the mixture is extracted with ethyl acetate. The organic layer is washed with dilute hydrochloric acid and then with water, dried over sodium sulfate and concentrated. The residue is purified by flash column chromatography using diisopropyl ether:chloroform:petroleum ether (1.5:4: 5.5) as eluent, pure 1α-t-butyldimethylsilyl-7β-t-buty laminocarbonyloxy-8,13-epoxy-6β,9α-dihydroxylabd-14-en-11-one being obtained in 64.6% yield. This is further treated by the procedure described in Example 2 with t-butylammonium fluoride trihydrate, there being obtained 7β-t-butylaminocarbonyloxy-8,13-epoxy-1α,6β,9α-trihydroxylabd-14-en-11-one of melting point 123-125 °C.

The following compounds are prepared in a similar manner:

7β-isopropylaminocarbonyloxy-8,13-epoxy-1α,6β,9α-trihydroxylabd-14-en-11-one, melting point 120-124 °C, and

7β-[N-Methyl-N-(N′-methylcarbamido]-aminocarbonyloxy-8,13-epoxy-1α,6β,9α-trihydroxy-labd-14-en-11-one, melting point 218-219 °C.

Example 4

7β-Cyclohexylaminocarbonyloxy-8,13-epoxy-1α,6β,9α-trihydroxylabd-14-en-11-one

Cyclohexyl isocyanate (1.5 ml, 12.2 mmol) is added to a solution of 1α-t-butyldimethylsilyl-8,13-epoxy-6β,7β,9α-trihydroxylabd-14-en-11-one (0.75 g, 1.56 mmol) in pyridine (5 ml), a catalytic amount of DMAP (0.05 g) is added and then the mixture is heated under reflux for 36 hours, poured onto ice, and the mixture is extracted with ethyl acetate. The organic layer is separated off, washed with dilute hydrochloric acid and then water, dried over sodium sulfate and concentrated. The residue is purified by flash column chromatography using chloroform:diisopropyl ether:petroleum ether (1:1:2) as eluent, and the pure product obtained in this way is subjected, under the conditions described in Example 2, to a treatment with tetrabutylammonium fluoride trihydrate, there being obtained 7β-cyclohexylaminocarbonyloxy-8,13-epoxy-1α,6β, 9α-trihydroxylabd-14-en-11-one.

The following compound is prepared in a similar manner:

7β-benzylaminothiocarbonyloxy-8,13-epoxy-1α,6β,9α-trihydroxylabd-14-en-11-one.

8,13-Epoxy-7β-methylaminocarbonyloxy-1α,6β,9α-trihydroxylabd-14-en-11-one.

Example 5

8,13-Epoxy-7β-m-trifluoromethylanilinocarbonyloxy-1α,6β, 9α-trihydroxylabd-14-en-11-one

m-Trifluoromethylphenyl isocyanate (0.19 ml, 1.38 mmol) is added to a solution of 8,13-epoxy-1α,6β,7β,9α-tetrahydroxylabd-14-en-11-one (0.5 g, 1.36 mmol) in pyridine (5 ml), and the mixture is stirred at room temperature for 10 hours and poured onto ice, and the mixture is extracted with ethyl acetate. The organic layer is separated off, washed with dilute hydrochloric acid and then with water, dried over anhydrous sodium sulfate and concentrated. The residue is purified by flash column chromatography using chloroform:diisopropyl ether:acetonitrile:petroleum ether (25:25:7:43) as eluent. The product resulting from this is crystallized from toluene:petroleum ether; melting point 139-142 °C.

The following compounds are prepared in a similar manner:
8,13-epoxy-7β-p-chloroanilinocarbonyloxy-1α,6β,9α-trihydroxylabd-14-en-11-one, melting point 194-195 °C.
7β-anilinocarbonyloxy-8,13-epoxy-1α,6β,9α-trihydroxylabd-14-en-11-one, melting point 168 °C.
8,13-epoxy-7β-p-toluenesulfonamidocarbonyloxy-1α,6β,9α-trihydroxylabd-14-en-11-one, melting point 184-185 °C, and
7β-benzenesulfonamidocarbonyloxy-8,13-epoxy-1α,6β,9α-trihydroxylabd-14-en-11-one, melting point 154-156 °C.

Similarly following compounds were prepared starting from 7β-acetoxy-8,13-epoxy-1α,6β,9α-trihydroxy-labd-14-en-11-one;
7β-Acetoxy-1α-cyclohexylaminocarbonyloxy-6β,9α-dihydroxy-8,13-epoxy-labd-14-en-11-one, m.p. 194-95 °C.
7β-Acetoxy-1α-anilinocarbonyloxy-6β,9α-dihydroxy-8,13-epoxy-labd-14-en-11-one, m.p. 223-25 °C.

Example 6

7β-Anilinocarbonyloxy-8,13-epoxy-1α,6β,9α-trihydroxylabd-14-en-11-one

Phenyl isocyanate (0.4 ml, 2.76 mmol) is added to a solution of 8,13-epoxy-1α,6β,7β,9α-tetrahy droxylabd-14-en-11-one (0.2 g, 0.55 mmol) in toluene (10 ml) or toluene (10 ml) containing pyridine (0.23 ml), and the mixture is left to stand at room temperature for 24 hours or is heated under reflux for 6 hours, following the reaction with TLC. The reaction mixture is then poured onto ice, the mixture is extracted with ethyl acetate, and the organic layer is washed with dilute hydrochloric acid and water, dried over anhydrous sodium sulfate and concentrated. The residue is purified by flash column chromatography using ethyl acetate:petroleum ether (3:7) as eluent, pure product of melting point 168 °C being obtained.

Example 7

8,13-Epoxy-7β-ethoxycarbonyloxy-1α,6β,9α-trihydroxylabd-14-en-11-one

Ethyl chloroformate (0.2 ml, 2.1 mmol) is added to a stirred, cooled solution of 8,13-epoxy-1α,6β,7β,9α-tetrahydroxylabd-14-en-11-one (0.2 g, 0.54 mmol) in pyridine (1.0 ml, 12.38 mmol), and the mixture is then stirred for a further 2 hours.

The reaction mixture is then diluted with ethyl acetate, washed with dilute hydrochloric acid, water and then brine, dried over anhydrous sodium sulfate and concentrated. The residue is purified by flash column chromatography using ethyl acetate:petroleum ether (2:8) as eluent, pure product of melting point 160-161 °C being obtained in 85% yield.

Example 8

8,13-Epoxy-7β-trichloroethoxycarbonyloxy-1α,6β,9α-trihydroxylabd-14-en-11-one

Trichloroethoxycarbonyl chloride (0.2 ml, 2 mmol) is added, under nitrogen, to a cooled solution of 8,13-epoxy-1α,6β, 7β,9α-tetrahydroxylabd-14-en-11-one (0.368 g, 1.0 mmol) in pyridine (5 ml), and the mixture is then stirred at room temperature (~ 28° C) for 16 hours.

The reaction mixture is diluted with ethyl acetate and washed with aqueous acetic acid and then water. The organic layer is separated off, dried over anhydrous sodium sulfate and concentrated. The residue is purified by flash column chromatography using ethyl acetate:petroleum ether (2:8) as eluent. The product obtained by this is crystallized from ethyl acetate:petroleum ether: yield 82.7%, melting point 169° C.

Example 9

6β-Anilino-carbonyloxy-8,13-epoxy-1α,7β,9α-trihydroxy-labd-14-en-11-one

Sodium methoxide (50 mg, 0.93 mmol) was added to a stirred solution of 7β-anilinocarbonyloxy-1α-t-butyldimethylsilyloxy-6β,9α-dihydroxy-8,13-epoxy-labd-14-en-11-one (0.5 g, 0.83 mmol) in dry dioxane (150 ml). The reaction mixture was stirred for 3 hrs and an aliquot of sodium methoxide (10 mg, 0.185 mmol) was added, stirring was continued for an hour. The reaction mixture was concentrated, extracted with ethylacetate. Organic layer was washed with water, dried over anhydrous sodium sulphate and concentrated. Residue was purified by using flash column chromatography using diisopropylether:chloroform:petroleum ether:triethylamine (30:50:20:0.5) mixture as an eluant. Product isolated was deprotected according to the procedure reported in example 2 to obtain 6β-anilino-carbonyloxy-8,13-epoxy-1α,7β,9α-trihydroxy-labd-14-en-11-one which was crystallized from ethyl acetate-petroleum ether. Yield 29 %, m.p. 169-172° C.

Example 10

7β-Acetoxy-1α-diethylaminocarbonyloxy-6β,9α-dihydroxy-8,13-epoxy-labd-14-en-11-one

Diethyl carbamyl chloride (1.18 ml, 9.3 mmol) was added to a mixture of 4-dimethylaminopyridine (0.10 g, 0.82 mmol) and 7β-acetoxy-8,13-epoxy-1α,6β,9α-trihydroxy-labd-14-en-11-one (1.0 g, 2.44 mmol) in dry pyridine (25 ml). Reaction mixture was refluxed for 72 hours, poured on ice and extracted with ethylacetate. Organic layer was washed with dilute HCl (10 % aq.) followed by water and brine, dried over anhydrous sodium sulphate and concentrated. Residue was purified by flash column chromatography using diisopropylether:chloroform: petroleum ether (2.5:5:2.5) as an eluant. Compound was crystallized from ethyl acetate:petroleum ether, yield 35 %, m.p. 158-159° C.

Similarly the following compound was prepared.

7β-Diethylamino-carbonyloxy-8,13-epoxy-1α,6β,9α-trihydroxy-labd-14-en-11-one, m.p. 216-219° C starting from 8.13-epoxy-1α,6β,7β,9α-tetrahydroxy-labd-14-en-11-one.

Example 11

8,13-Epoxy-7β-methylaminothiocarbonyloxy-1α,6β,9α-trihydroxy-labd-14-en-11-one

1,1′-Thiocarbonyl-diimidazole (1 g, 5.62 mmol) was added to a stirred solution of 1α-t-butyl-dimethylsilyloxy-8,13-epoxy-6β,7β,9α-trihydroxy-labd-14-en-11-one (0.45 g, 0.93 mmol) in dry ethyl acetate (25 ml) under nitrogen atmosphere. The reaction mixture was stirred overnight at room temperature and another aliquot of 1,1′-thiocarbonyl-diimidazole (1 g (5.62 mmol) was added to the reaction mixture and stirring was

continued for another 24 hours at room temperature.

Methylamine in toluene (10 ml, conc. 25 ml in 125 ml toluene) was added to the above reaction mixture and stirring was continued for 1 hour. The reaction mixture was diluted with ethyl acetate and filtered. The filtrate was washed with 10 % HCl, brine, dried over anhydrous sodium sulphate and concentrated. Residue was purified by flash column chromatography using ethyl acetate: petroleum ether (2:8) as an eluant to obtain the product in 14.5 % yield. Deprotection carried out according to example 2 gave 8,13-epoxy-7β-methylamino-thiocarbonyloxy-1α,6β,9α-trihydroxy-labd-14-en-11-one.
Yield 90 %, m.p. 223-224° C.

Similarly the following compound was prepared:
7β-Benzylaminothiocarbonyloxy-8,13-epoxy-1α,6β,9α-trihydroxy-labd-14-en-11-one, yield 40 %, m.p. 204-208° C.

## Claims

1. A compound of the formula I

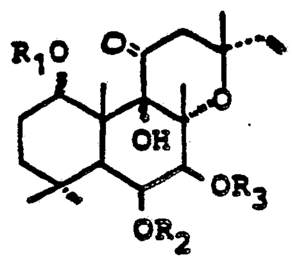

I

in which $R_1$, $R_2$ and $R_3$ are identical or different and denote:
hydrogen, $C_1$-$C_6$-alkyl, $C_2$-$C_6$-alkanoyl or a radical of the formula
$-\overset{Z}{\overset{\|}{C}}-A$, in which Z represents oxygen or sulfur, and A either represents the radical

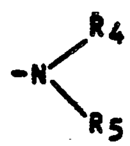

in which $R_4$ represents hydrogen or $C_1$-$C_6$-alkyl, and $R_5$ represents $C_1$-$C_6$-alkyl, $C_3$-$C_7$-cycloalkyl, aryl, aryl-$C_1$-$C_6$-alkyl, $C_2$-$C_6$-carbalkoxy or sulfonylaryl, or $R_4$ and $R_5$, together with the nitrogen atom to which they are bonded, form a heterocycle which may contain as further heteroatom oxygen, nitrogen or sulfur, or A represents the radical $-OR_6$ in which $R_6$ represents $C_1$-$C_6$-alkyl or halogeno-$C_1$-$C_6$-alkyl, $R_1$ represents a tris($C_1$-$C_6$-alkyl)silyl group, and $R_2$ and $R_3$ have the said meanings,
with the proviso that
1. $R_1$, $R_2$ and $R_3$ do not simultaneously represent hydrogen, and
2. at least one of the three substituents $R_1$, $R_2$ and $R_3$ represent the radical $-\overset{Z}{\overset{\|}{C}}-A$ when the other substituent(s) represent(s) hydrogen, $C_1$-$C_6$-alkyl or $C_1$-$C_6$-alkanoyl,
and its pharmacologically acceptable acid addition salts and optical and geometric isomers.

2. A process for the preparation of a compound as claimed in claim 1, which comprises reacting a compound of the formula II

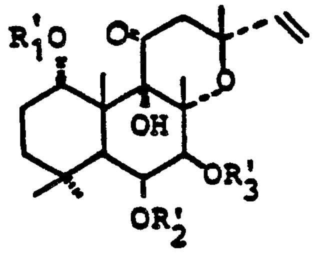

II

in which $R_1'$, $R_2'$ and $R_3'$ denote hydrogen, or $R_1'$ and $R_2'$ denote hydrogen, and $R_3'$ denotes the acetyl group, or $R_1'$ denotes a lower alkylsilyl group, and $R_2'$ and $R_3'$ denote hydrogen,

a) initially with a compound of the formula III

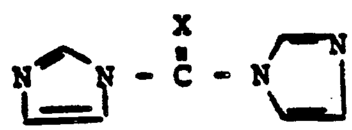

III

in which X denotes oxygen or sulfur, and then with an amine of the formula

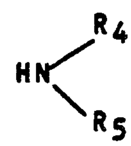

in which $R_4$ and $R_5$ have the said meaning, or

b) with an isocyanate of the formula RNCX, in which R denotes cycloalkyl or aryl and X denotes oxygen or sulfur, where appropriate eliminating the alkylsilyl group from the 1-position, and isolating the compound of the formula I from the reaction mixture in a customary manner.

3. A medicament which contains a compound as claimed in claim 1, besides pharmaceutically customary auxiliaries and/or vehicles.

4. The use of a compound as claimed in claim 1 for preparing a medicament acting to lower the intraocular pressure.

Claims for the following contracting States: ES, GR

1. A process for the preparation of a compound of the formula I

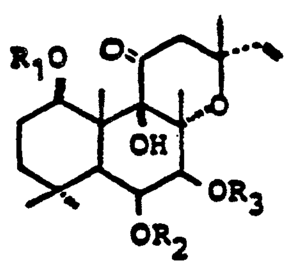

I

in which $R_1$, $R_2$ and $R_3$ are identical or different and denote:
hydrogen, $C_1$-$C_6$-alkyl, $C_2$-$C_6$-alkanoyl or a radical of the formula
$-\overset{Z}{\overset{\parallel}{C}}$-A, in which Z represents oxygen or sulfur, and A either represents the radical

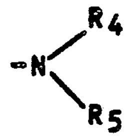

in which $R_4$ represents hydrogen or $C_1$-$C_6$-alkyl, and $R_5$ represents $C_1$-$C_6$-alkyl, $C_3$-$C_7$-cycloalkyl, aryl, aryl-$C_1$-$C_6$-alkyl, $C_2$-$C_6$-carbalkoxy or sulfonylaryl, of $R_4$ and $R_5$, together with the nitrogen atom to which they are bonded, form a heterocycle which may contain as further heteroatom oxygen, nitrogen or sulfur, or A represents the radical -$OR_6$ in which $R_6$ represents $C_1$-$C_6$-alkyl or halogeno-$C_1$-$C_6$-alkyl, $R_1$ represents a tris($C_1$-$C_6$-alkyl)silyl group, and $R_2$ and $R_3$ have the said meanings, with the proviso that
1. $R_1$, $R_2$ and $R_3$ do not simultaneously represent hydrogen, and
2. at least one of the three substituents $R_1$, $R_2$ and $R_3$ represent the radical $-\overset{\overset{Z}{\|}}{C}-A$ when the other substituent(s) represent(s) hydrogen, $C_1$-$C_6$-alkyl or $C_1$-$C_6$-alkanoyl,
and its pharmacologically acceptable acid addition salts and optical and geometric isomers,

which comprises reacting a compound of the formula II

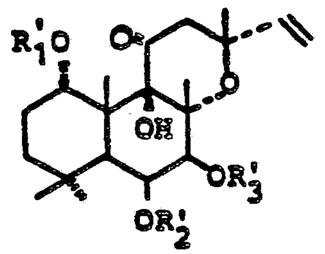

II

in which $R_1'$, $R_2'$ and $R_3'$ denote hydrogen, or $R_1'$ and $R_2'$ denote hydrogen, and $R_3'$ denotes the acetyl group, or $R_1'$ denotes a lower alkylsilyl group, and $R_2'$ and $R_3'$ denote hydrogen,

a) initially with a compound of the formula III

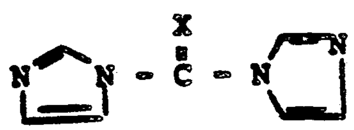

III

in which X denotes oxygen or sulfur, and then with an amine of the formula

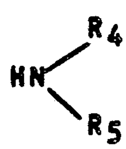

in which $R_4$ and $R_5$ have the said meaning, or

b) with an isocyanate of the formula RNCX, in which R denotes cycloalkyl or aryl and X denotes oxygen or sulfur, where appropriate eliminating the alkylsilyl group from the 1-position, and isolating the compound of the formula I from the reaction mixture in a customary manner.

2. The use of a compound as claimed in claim 1 for preparing a medicament acting to lower the intraocular pressure.